# EUROPEAN PATENT APPLICATION

(11) **EP 3 415 096 A1**
(43) Date of publication of application: **19.12.2018**
(21) Application number: 17750177.2
(22) Date of filing: 03.02.2017
(51) Int. Cl.: A61B 8/08

(54) **ULTRASONIC OBSERVATION DEVICE, OPERATION METHOD FOR ULTRASONIC OBSERVATION DEVICE, AND OPERATION PROGRAM FOR ULTRASONIC OBSERVATION DEVICE**

(30) Priority: 12.02.2016 JP 2016025217
(71) Applicant: Olympus Corporation, Hachioji-shi Tokyo 192-8507 (JP)
(72) Inventor: MIYAKE, Tatsuya, Hachioji-shi Tokyo 192-8507 (JP)
(74) Representative: Gunzelmann, Rainer
(86) International application number: PCT/JP2017/003973
(87) International publication number: WO 2017/138454

(57) **Abstract**

An ultrasound observation apparatus includes: an ultrasound image generator that generates data of an ultrasound image based on an ultrasound wave signal; a region of interest setting unit that sets a first region of interest set in advance within the ultrasound image and a plurality of second regions of interest including at least the first region of interest, when receiving an input of a freeze instruction signal that sets a display on a display device as a still image; a reference value calculating unit that calculates reference values according to ultrasound wave signals of the first region of interest and the plurality of second regions of interest, respectively; and an elastic image generator that sets a display region of interest in which the reference value of the first region of interest and the reference values of the second regions of interest have a predetermined correlation and the second regions of interest are maximized, and generates data of an elastic image having a display mode according to hardness of the display region of interest. As a result, in ultrasound elastography, an ultrasound observation apparatus capable of expanding a colored area of the elastic image while suppressing the influence on a color tone of the elastic image within the region of interest set by a user is provided.

## Description

### Field

The present invention relates to an ultrasound observation apparatus for observing a tissue of an observation object using an ultrasound wave, an operation method for an ultrasound observation apparatus, and an operation program for an ultrasound observation apparatus. Background

Conventionally, ultrasound elastography is known as a technology for diagnosing an observation object using an ultrasound wave (for example, see Patent Literature 1). The ultrasound elastography is a technique that uses the fact that hardness of a cancer or tumor tissue in a living body is different depending on a progress condition of disease or the living body. In this technology, an average value of displaced amounts of a biological tissue in a predetermined region of interest (ROI) is colored as a reference value, thereby generating an elastic image in which information on the hardness of the biological tissue is imaged.

In the ultrasound elastography, a user sets the region of interest according to observation contents. In the conventional ultrasound elastography, it is common that only the region of interest set by the user is colored.

### Citation List

### Patent Literature

Patent Literature 1: Japanese Patent No. 5465671 Summary

### Technical Problem

Also, it is preferable to be able to know information on the hardness of the biological tissue in an area as wide as possible when a user such as a doctor executes a diagnosis using the ultrasound elastography. However, when the area to be colored is expanded from the region of interest to a wider area, the reference value that is the average value of the displaced amounts in the area is changed. As a result, there is a problem that it is difficult for a user to observe a change in a color tone of the entire elastic image.

An object of the present invention is to provide an ultrasound observation apparatus capable of expanding a colored area of an elastic image while suppressing an influence on a color tone of an elastic image within a region of interest set by a user, in ultrasound elastography, an operation method for an ultrasound observation apparatus, and an operation program for an ultrasound observation apparatus.

### Solution to Problem

To solve the problem described above and to achieve the object, an ultrasound observation apparatus according to one aspect of the present invention is an ultrasound observation apparatus performing an observation based on an ultrasound wave signal acquired by an ultrasound probe including an ultrasound transducer transmitting an ultrasound wave to an observation object and receiving the ultrasound wave reflected from the observation object. The ultrasound observation apparatus includes: an ultrasound image generator configured to generate data of an ultrasound image based on the ultrasound wave signal; a region of interest setting unit configured to set a first region of interest set in advance within the ultrasound image and a plurality of second regions of interest including at least the first region of interest, when receiving an input of a freeze instruction signal that sets a display on a display device as a still image; a reference value calculating unit configured to calculate reference values according to ultrasound wave signals of the first region of interest and the plurality of second regions of interest, respectively; and an elastic image generator configured to set a display region of interest in which the reference value of the first region of interest and the reference values of the second regions of interest have a predetermined correlation and the second regions of interest are maximized, and generate data of an elastic image having a display mode according to hardness of the display region of interest.

In the ultrasound observation apparatus according to one aspect of the present invention, the predetermined correlation is a relationship in which a ratio or a difference between the reference value of the first region of interest and the reference values of the second regions of interest falls within a predetermined range.

In the ultrasound observation apparatus according to one aspect of the present invention, the region of interest setting unit is configured to successively set the second regions of interest by excluding a part of the second regions of interest having a maximum area within the ultrasound image from the second regions of interest having the maximum area within the ultrasound image according to a predetermined rule, the reference value calculating unit is configured to successively calculate the reference values of the second regions of interest that the region of interest setting unit has set, and the elastic image generator is configured to successively compare the reference value of the first region of interest with the reference values of the second regions of interest, and when the reference value of the first region of interest and the reference values of the second regions of interest have the predetermined correlation, set the second regions of interest at that time as the display region of interest.

In the ultrasound observation apparatus according to one aspect of the present invention, the region of interest setting unit is configured to set the second regions of interest by sequentially excluding an area farther from the first region of interest.

In the ultrasound observation apparatus according to one aspect of the present invention, the region of interest setting unit is configured to set the second regions of interest by sequentially excluding an area along a sound ray of the ultrasound transducer constituting the ultrasound image and an area along a scanning direction of the ultrasound transducer constituting the ultrasound image.

The ultrasound observation apparatus according to one aspect of the present invention further includes: an average image generator configured to generate, when receiving an input of an average instruction signal for generating an average image obtained by adding up and averaging a plurality of ultrasound images, data of the average image. The region of interest setting unit is configured to set the first region of interest and the plurality of second regions of interest in the respective ultrasound images of the plurality of ultrasound images, and the elastic image generator is configured to set an area, in which the reference value of the first region of interest in the respective ultrasound images and the reference values of the second regions of interest in the respective ultrasound images have the predetermined correlation and the second regions of interest are maximized, as third regions of interest, and set an area within the average image corresponding to an area in which the respective third regions of interest overlap with each other, as the display region of interest.

In the ultrasound observation apparatus according to one aspect of the present invention, the region of interest setting unit is configured to set the second regions of interest obtained by expanding a part of the first region of interest from the first region of interest according to a predetermined rule.

In the ultrasound observation apparatus according to one aspect of the present invention, the predetermined correlation is a relationship in which a difference between the reference value of the first region of interest and the reference values of the second regions of interest falls within a predetermined range, and the elastic image generator is configured to change the predetermined range according to the reference value of the first region of interest.

In the ultrasound observation apparatus according to one aspect of the present invention, the reference value is a statistic corresponding to the ultrasound wave signal.

An operation method for an ultrasound observation apparatus according to one aspect of the present invention is an operation method for an ultrasound observation apparatus performing an observation based on an ultrasound wave signal acquired by an ultrasound probe including an ultrasound transducer transmitting an ultrasound wave to an observation object and receiving the ultrasound wave reflected from the observation object. The operation method includes: a region of interest setting step of setting, by a region of interest setting unit, a first region of interest set in advance within an ultrasound image generated based on the ultrasound wave signal and a plurality of second regions of interest including at least the first region of interest, when an input of a freeze instruction signal that sets a display on a display device as a still image is received; a reference value calculation step of calculating, by a reference value calculating unit, reference values according to ultrasound wave signals of the first region of interest and the plurality of second regions of interest, respectively; and an elastic image generation step of setting, by an elastic image generator, a display region of interest in which the reference value of the first region of interest and the reference values of the second regions of interest have a predetermined correlation and the second regions of interest are maximized, and generating data of an elastic image having a display mode according to hardness of the display region of interest.

An operation program for an ultrasound observation apparatus according to one aspect of the present invention is an operation program for an ultrasound observation apparatus causing the ultrasound observation apparatus performing an observation based on an ultrasound wave signal acquired by an ultrasound probe including an ultrasound transducer transmitting an ultrasound wave to an observation object and receiving the ultrasound wave reflected from the observation object to execute the following steps: a region of interest setting step of setting, by a region of interest setting unit, a first region of interest set in advance within an ultrasound image generated based on the ultrasound wave signal and a plurality of second regions of interest including at least the first region of interest, when an input of a freeze instruction signal that sets a display on a display device as a still image is received; a reference value calculation step of calculating, by a reference value calculating unit, reference values according to ultrasound wave signals of the first region of interest and the plurality of second regions of interest, respectively; and an elastic image generation step of setting, by an elastic image generator, a display region of interest in which the reference value of the first region of interest and the reference values of the second regions of interest have a predetermined correlation and the second regions of interest are maximized, and generating data of an elastic image having a display mode according to hardness of the display region of interest.

### Advantageous Effects of Invention

According to the present invention, it is possible to realize the ultrasound observation apparatus capable of expanding the colored area of the elastic image while suppressing the influence on the color tone of the elastic image within the region of interest set by the user, in the ultrasound elastography, the operation method for an ultrasound observation apparatus, and the operation program for an ultrasound observation apparatus.

### Brief Description of Drawings

FIG. 1 is a diagram schematically illustrating a configuration of an ultrasound diagnostic system including an ultrasound observation apparatus according to a first embodiment of the present invention.
FIG. 2 is a flowchart illustrating an outline of processing performed by the ultrasound observation apparatus according to the first embodiment of the present invention.
FIG. 3 is a diagram illustrating an example in which an elastic image within a first ROI is displayed on a display device.
FIG. 4 is a diagram illustrating displaced amounts of each pixel.
FIG. 5 is a diagram illustrating an example of a method for setting second ROIs.
FIG. 6 is a diagram illustrating an example of a method for setting second ROIs.
FIG. 7 is a diagram illustrating an example of a method for setting second ROIs.
FIG. 8 is a diagram illustrating an example of a method for setting second ROIs.
FIG. 9 is a diagram illustrating an example in which an elastic image within a display region of interest is displayed on the display device.
FIG. 10 is a diagram schematically illustrating a configuration of an ultrasound diagnostic system including an ultrasound observation apparatus according to a second embodiment of the present invention.
FIG. 11 is a flowchart illustrating an outline of processing performed by the ultrasound observation apparatus according to the second embodiment of the present invention.
FIG. 12 is a flowchart illustrating an outline of processing performed by an ultrasound observation apparatus according to a third embodiment of the present invention.
FIG. 13 is a diagram illustrating an example of a method for setting second ROIs.
FIG. 14 is a diagram illustrating an example of a method for setting second ROIs.
FIG. 15 is a diagram illustrating an example of a method for setting second ROIs.
FIG. 16 is a diagram illustrating an example of a method for setting second ROIs.

### Description of Embodiments

Hereinafter, a mode (hereinafter referred to as "embodiment") for carrying out the present invention will be described with reference to the accompanying drawings.

### (First Embodiment)

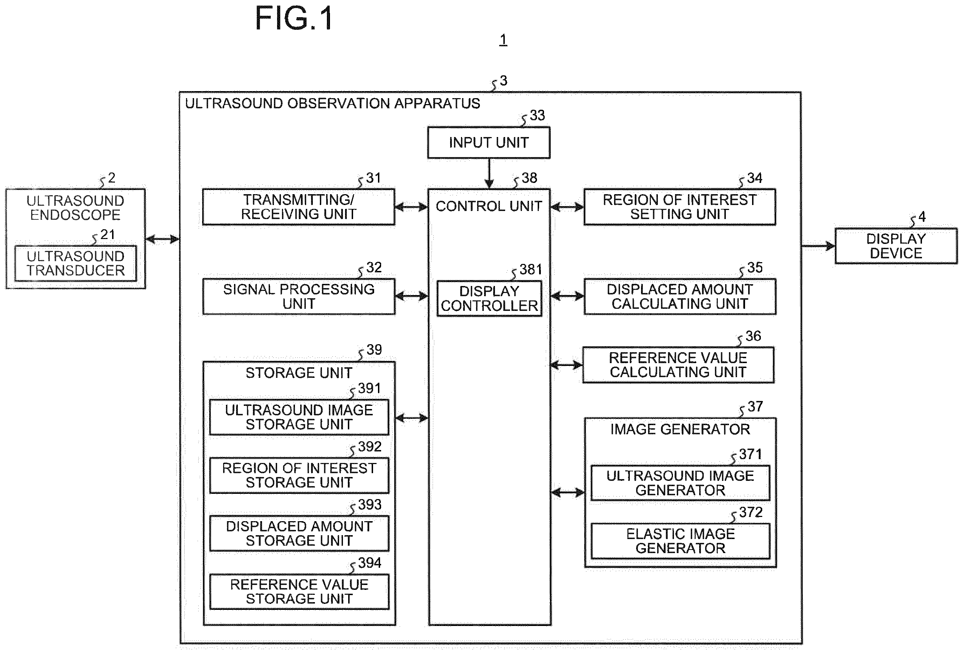

FIG. 1 is a diagram schematically illustrating a configuration of an ultrasound diagnostic system including an ultrasound observation apparatus according to a first embodiment of the present invention. An ultrasound diagnostic system 1 illustrated in FIG. 1 includes an ultrasound endoscope 2, an ultrasound observation apparatus 3, and a display device 4.

The ultrasound endoscope 2 has an ultrasound transducer 21 that is provided at a distal end portion of the ultrasound endoscope 2 and transmits an ultrasound wave to a subject that is an observation object and receives the ultrasound wave reflected from the subject. The ultrasound transducer 21 converts an electrical pulse signal received from the ultrasound observation apparatus 3 into an ultrasound pulse (acoustic pulses) and irradiates the subject with the ultrasound pulse, and converts an ultrasound echo reflected from the subject into an electrical echo signal (ultrasound wave signal) and outputs the electrical echo signal. The ultrasound transducer 21 may be an electronic scanning type or a mechanical scanning type. It is known that a type of ultrasound endoscope 2 is various according to observation objects such as a digestive tract (esophagus, stomach, duodenum, and large intestine) and a respiratory system (trachea and bronchus) of the subject.

The ultrasound endoscope 2 may further include an imaging unit that images an inside of the subject and a light guide that guides illumination light from a light source device, which generates the illumination light to be irradiated onto the subject at the time of imaging, to the distal end of the ultrasound endoscope 2.

The ultrasound observation apparatus 3 transmits and receives an electrical signal to and from the ultrasound endoscope 2 via an ultrasound cable. The ultrasound observation apparatus 3 performs predetermined processing on the electrical echo signal received from the ultrasound endoscope 2 to generate an ultrasound image or the like. The ultrasound observation apparatus 3 includes a transmitting/receiving unit 31 that transmits and receives a signal to and from the ultrasound transducer 21, a signal processing unit 32 that generates digital reception data based on an echo signal received from the transmitting/receiving unit 31, an input unit 33 that receives an input of various information including an operation instruction signal of the ultrasound observation apparatus 3, a region of interest setting unit 34 that sets a region of interest in an ultrasound image, a displaced amount calculating unit 35 that calculates a displaced amount between images at an observation point (sampling point) in the region of interest, a reference value calculating unit 36 that calculates a reference value based on the displaced amount, an image generator 37 that generates data of various images including an ultrasound image and an elastic image, a control unit 38 that integrally controls the overall operation of the ultrasound diagnostic system 1, and a storage unit 39 that stores various information required for the operation of the ultrasound observation apparatus 3. The ultrasound observation apparatus 3 can set an elastography mode in which information on relative hardness of the observation object in the region of interest is imaged and expressed by visual information such as color.

The transmitting/receiving unit 31 transmits a pulse-shaped transmission drive wave signal to the ultrasound transducer 21 based on a predetermined waveform and transmission timing. In addition, the transmitting/receiving unit 31 receives the electrical echo signal from the ultrasound transducer 21. The transmitting/receiving unit 31 serves to transmit various control signals output from the control unit 38 to the ultrasound endoscope 2 and receive various information including an ID for identification from the ultrasound endoscope 2 and transmit the received information to the control unit 38.

The signal processing unit 32 performs known processing, such as a band pass filtering, envelope detection, and logarithmic conversion, on the echo signal to generate received data for digital ultrasound image (hereinafter, referred to as received data). The signal processing unit 32 is realized by using a general-purpose processor such as a central processing unit (CPU), a dedicated integrated circuit for executing a specific function such as an application specific integrated circuit (ASIC) or a field programmable gate array (FPGA), or the like.

The input unit 33 receives an input of a signal that instructs setting of a first region of interest (hereinafter referred to as a first ROI). In addition, the input unit 33 receives an input of a freeze instruction signal that sets a display on the display device 4 as a still image. The input unit 33 is configured by using a user interface such as a keyboard, a mouse, and a touch panel.

The region of interest setting unit 34 sets the first ROI based on a setting input received by the input unit 33. In addition, if the input unit 33 receives the input of the freeze instruction signal, the region of interest setting unit 34 sets a plurality of second regions of interest (hereinafter referred to as a second ROI) including the first ROI.

The displaced amount calculating unit 35 calculates a displaced amount of a tissue at an observation point (sampling point) in the ultrasound image according to pressurization by a pulsation of the subject based on the received data generated by the signal processing unit 32. The displaced amount calculating unit 35 calculates the displaced amount by comparing, for example, the latest ultrasound image with the ultrasound image generated one frame before.

The reference value calculating unit 36 calculates a reference value corresponding to the displaced amount calculated by the displaced amount calculating unit 35 of the first ROI and the plurality of second ROIs, respectively. The reference value is, for example, an average value (statistic) of the displaced amounts in the first ROI and the plurality of second ROIs. However, the reference value may be a statistic such as a maximum value, a minimum value, a median value, and a mode value.

The image generator 37 includes an ultrasound image generator 371 that generates data of the ultrasound image based on the received data and an elastic image generator 372 that generates an elastic image visually expressing the information on the hardness of the tissue of the observation object based on the displaced amount in the ultrasound image.

The data of the ultrasound image generated by the ultrasound image generator 371 is, for example, B mode image data obtained by converting amplitude into luminance.

The elastic image generator 372 sequentially generates data of an elastic image having a display mode corresponding to the hardness of the first ROI until the input unit 33 receives the input of the freeze instruction signal. If the input unit 33 receives the input of the freeze instruction signal, the elastic image generator 372 sets the display region of interest in which the reference value of the first ROI and the reference values of the second ROIs have a predetermined correlation and the second ROIs are maximized. The predetermined correlation is, for example, a relationship in which a difference between the reference value of the first ROI and the reference values of the second ROIs falls within a predetermined range, but may be a relationship having a predetermined correlation such as a ratio, a sum, and a product of the reference value of the first ROI and the reference values of the second ROIs. The elastic image generator 372 generates the data of the elastic image having the display mode corresponding to the hardness of the display region of interest. The elastic image generated by the elastic image generator 372 is an image obtained by assigning visual information such as color and pattern to each point in the display region of interest based on the calculation result of the displaced amount calculating unit 35. Specifically, the elastic image generator 372 generates the data of the elastic image by assigning green to a tissue corresponding to the average hardness in the display region of interest, color of a blue tone to a tissue harder than the average, and color of a red tone to a tissue softer than the average.

The control unit 38 has a display controller 381 that controls the display on the display device 4. The display controller 381 controls the display device 4 to display various images generated by the image generator 37.

The control unit 38 is realized by using a general-purpose processor such as a CPU having calculation and control functions, or a dedicated integrated circuit such as ASIC or FPGA. In the case where the control unit 38 is realized by the general-purpose processor or the FPGA, various programs or various data stored in the storage unit 39 are read from the storage unit 39 and various calculation processings related to the operation of the ultrasound observation apparatus 3 are executed, thereby integrally controlling the ultrasound observation apparatus 3. In the case where the control unit 38 is configured using the ASIC, various processings may be performed independently, or various processings may be executed by using various data or the like that is stored in the storage unit 39. In the first embodiment, the control unit 38, and at least a part of the signal processing unit 32, the region of interest setting unit 34, the displaced amount calculating unit 35, the reference value calculating unit 36, and the image generator 37 may be configured by a common general-purpose processor, a dedicated integrated circuit, or the like.

The storage unit 39 includes an ultrasound image storage unit 391 that at least temporarily stores data of a plurality of ultrasound images generated by the ultrasound image generator 371, a region of interest storage unit 392 that stores information on the first ROI and the second ROIs set for the ultrasound image, a displaced amount storage unit 393 that stores the displaced amount calculated by the displaced amount calculating unit 35, and a reference value storage unit 394 that stores the reference value calculated by the reference value calculating unit 36. The number of ultrasound images stored in the ultrasound image storage unit 391 is preset. The displaced amount storage unit 393 stores data of the displaced amount including the amount that is required when the displaced amount calculating unit 35 calculates the displaced amount. The reference value storage unit 394 stores data of the reference value including the amount that is required when the region of interest setting unit 34 performs the comparison of the reference value.

The storage unit 39 stores various programs including an operation program for executing the operation method for the ultrasound observation apparatus 3. Various programs including the operation program can be recorded on computer readable recording media such as a hard disk, a flash memory, a CD-ROM, a DVD-ROM, and a flexible disk and distributed widely. It should be noted that the above-described various programs can also be acquired by being downloaded via a communication network. The communication network referred herein is realized by, for example, an existing public network, a local area network (LAN), a wide area network (WAN), and the like, and may be wired or wireless.

The storage unit 39 is realized by using a read only memory (ROM) in which various programs and the like are installed in advance, a random access memory (RAM) in which calculation parameters, data or the like of each processing is stored, and the like.

The display device 4 is configured using liquid crystal, organic electro luminescence (EL), or the like, receives image data such as an ultrasound image and an elastic image generated by the ultrasound observation apparatus 3, and displays these images.

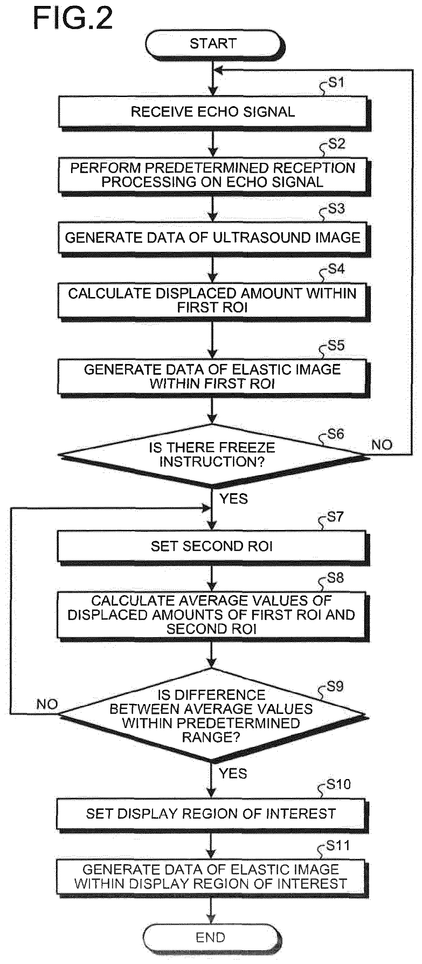

FIG. 2 is a flowchart illustrating an outline of processing performed by the ultrasound observation apparatus according to the first embodiment of the present invention. The flowchart illustrated in FIG. 2 illustrates processing in the case in which the ultrasound diagnostic system 1 is set in the elastography mode, the transmitting/receiving unit 31 starts to transmit the transmission drive wave, the ultrasound transducer 21 starts to transmit the ultrasound wave and then the setting of the first ROI in the ultrasound image is completed.

First, the transmitting/receiving unit 31 receives the echo signal that is the measurement result of the observation object by the ultrasound transducer 21 from the ultrasound endoscope 2 (step S1).

Subsequently, the signal processing unit 32 generates received data by performing predetermined reception processing on the echo signal received from the ultrasound transducer 21 (step S2). Specifically, the transmitting/receiving unit 31 amplifies the echo signal (STC correction), and then performs processings such as filtering and A/D conversion.

Thereafter, the ultrasound image generator 371 generates the data of the ultrasound image using the received data generated by the signal processing unit 32, stores the generated data in the ultrasound image storage unit 391, and outputs the data to the display device 4 under the control of the display controller 381 (step S3).

Subsequently, the displaced amount calculating unit 35 calculates the displaced amount at the observation point in the first ROI stored in the region of interest storage unit 392 (step S4). At this time, the displaced amount calculating unit 35 calculates a displaced amount at each observation point by using data of the latest ultrasound image and data of the past ultrasound image stored in the ultrasound image storage unit 391.

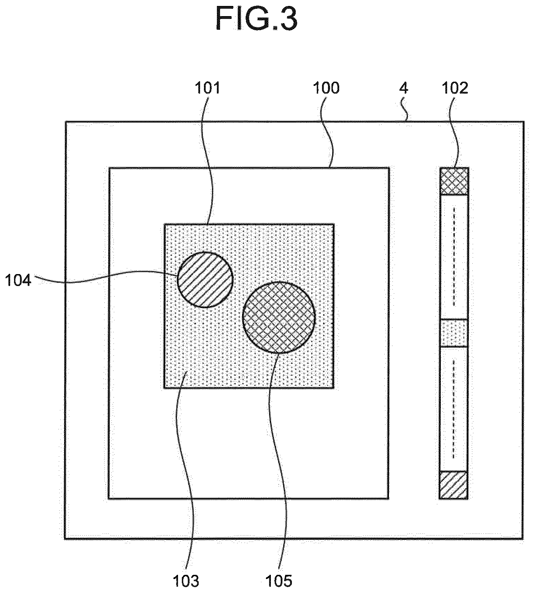

Thereafter, the elastic image generator 372 generates the data of the elastic image within the first ROI using the calculation results of the displaced amounts at each observation point in step S4, and outputs the generated data to the display device 4 (step S5). FIG. 3 is a diagram illustrating the example in which the elastic image within the first ROI is displayed on the display device. An elastic image 100 illustrated in FIG. 3 is displayed by identifiably imaging hardness of each tissue within a first ROI 101 by color. In FIG. 3, color differences are schematically represented by patterns. A color scale 102 which represents color used in the elastic image 100 is displayed on the right side of the first ROI 101. The color scale 102 corresponds to a state in which a tissue is hardened according to an upper color. In FIG. 3, only a part of the color scale 102 is schematically illustrated. An area 103 illustrated in FIG. 3 is an area in which the displaced amount is average. On the other hand, an area 104 is the relatively softest area, and an area 105 is the relatively hardest area. The display device 4 displays the elastic image 100 in parallel with a B mode image. The display device 4 may display the elastic image 100 by superimposing the elastic image 100 on the B mode image. In this case, the image generator 37 generates data of an image in which the elastic image 100 is superimposed on the B mode image, and outputs the data of the image to the display device 4 under the control of the display controller 381.

In this case, the control unit 38 determines whether the input unit 33 receives the instruction input of the freeze instruction signal (step S6). As the determination result, when the instruction input of the freeze instruction signal is received (step S6: Yes), the processing proceeds to step S7. On the other hand, as the determination result, when the instruction input of the freeze instruction signal is not received (step S6: No), the processing returns to step S1, and the processings of steps S1 to S5 are repeatedly performed.

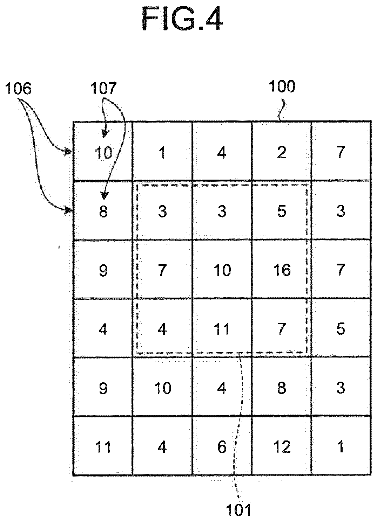

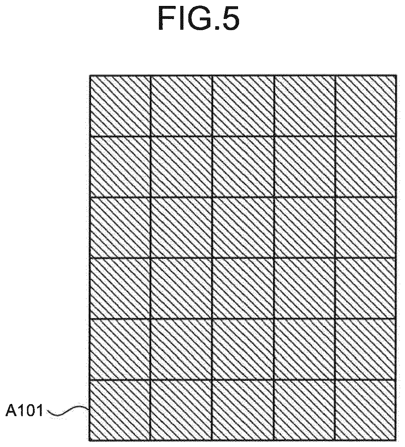

In step S7, the region of interest setting unit 34 sets the second ROIs. FIG. 4 is a diagram illustrating the displaced amounts of each pixel. In FIG. 4, a pixel 106 arranged in a two-dimensional shape and displaced amounts 107 of the pixel 106 are schematically illustrated. An area surrounded by a broken line in a center of FIG. 4 corresponds to the first ROI 101. In this case, the method for setting second ROIs based on the displaced amounts 107 of each pixel 106 is described, but the second ROIs may be set based on the average, the sum or the like of the displaced amounts 107 of a plurality of pixel groups. FIG. 5 is a diagram illustrating an example of the method for setting second ROIs. First, as illustrated in FIG. 5, the region of interest setting unit 34 sets an area A101, which includes all the pixels 106 illustrated in FIG. 4, in the second ROIs so that the second ROIs are maximized.

Subsequently, the reference value calculating unit 36 calculates the average value of the displaced amounts 107 in the first ROI 101 and the second ROIs (step S8). Specifically, the reference value calculating unit 36 calculates 7.33 as an average value by dividing a sum (66) of the displaced amounts 107 in the first ROI 101 by the number (9) of pixels included in the first ROI 101. In addition, the reference value calculating unit 36 calculates 6.47 as an average value by dividing a sum (194) of the displaced amounts 107 in the second ROIs as the area A101 by the number (30) of pixels included in the first ROI 101.

Thereafter, the elastic image generator 372 determines whether the difference between the average value of the displaced amounts 107 in the first ROI 101 and the average value of the displaced amounts 107 in the second ROIs falls within a predetermined range under the control of the display controller 381 (step S9). Specifically, for example, it is determined whether the difference between the average value of the displaced amounts 107 in the first ROI 101 and the average value of the displaced amounts 107 in the second ROIs falls within a range of -0.01 to 0.01. In this case, since the difference (0.87) between the average value of the displaced amounts 107 in the first ROI 101 and the average value of the displaced amounts 107 in the second ROIs is greater than 0.01 (step S9: No), the processing returns to step S7 to set new second ROIs.

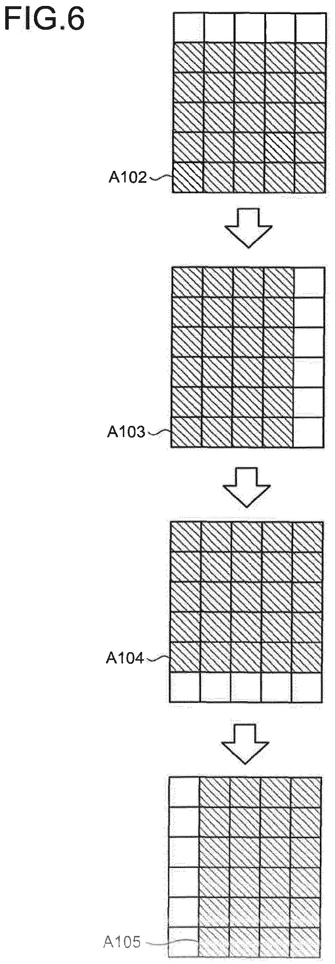

FIGS. 6 to 8 are diagrams illustrating an example of the method for setting second ROIs. As illustrated in FIGS. 6 to 8, the region of interest setting unit 34 successively sets the second ROIs by excluding a part of the area from the area A101 of FIG. 5 that is the second ROIs having the largest area within the ultrasound image according to a predetermined rule. Specifically, the region of interest setting unit 34 sets the second ROIs by sequentially excluding an area along a sound ray direction (corresponding to a vertical direction in FIGS. 6 to 8) of the ultrasound transducer 21 constituting the ultrasound image and an area along a scanning direction (corresponding to a horizontal direction in FIGS. 6 to 8) of the ultrasound transducer 21 constituting the ultrasound image.

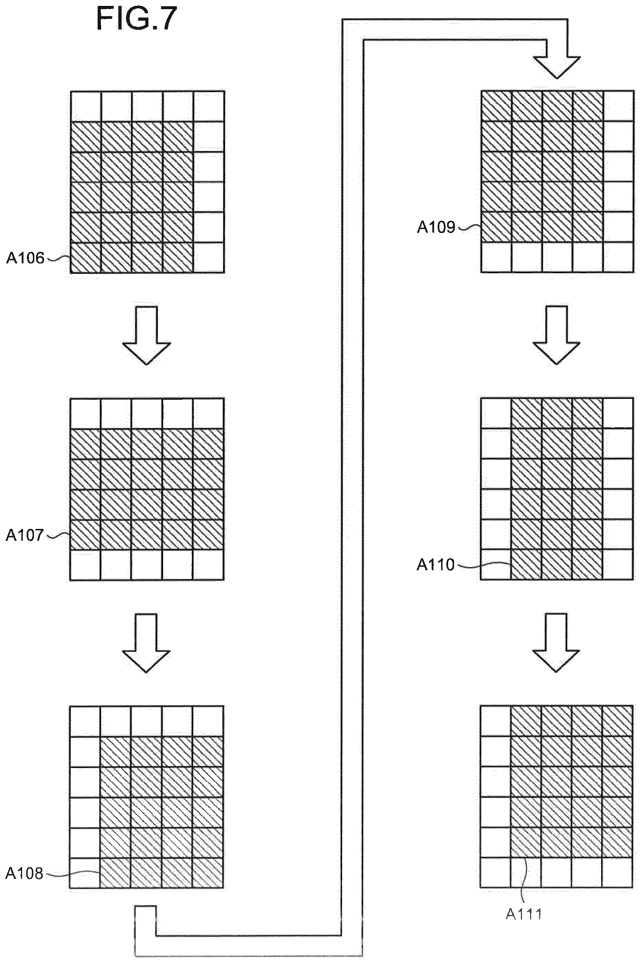

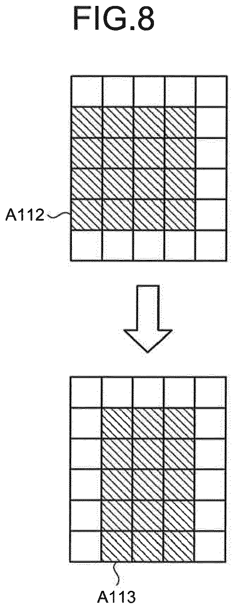

An area A102 to an area A105 in FIG. 6 are areas in which one pixel is excluded from the area A101 in any one of up, down, left, and right directions. An area A106 to an area A111 in FIG. 7 are areas in which one pixel is excluded from the area A101 in two of up, down, left, and right directions. An area A112 and an area A113 in FIG. 8 are areas in which one pixel is excluded from the area A101 in three of up, down, left, and right directions. In this way, the region of interest setting unit 34 successively sets the areas A102 to A113 in the second ROIs.

In step S8, the reference value calculating unit 36 successively calculates the average value of the displaced amounts 107 in the second ROIs set by the region of interest setting unit 34. In addition, in step S9, the elastic image generator 372 determines whether the difference between the average value of the displaced amounts 107 in the first ROI 101 and the average value of the displaced amounts 107 in the second ROIs falls within a predetermined range under the control of the display controller 381. In this case, when the second ROIs are an area A102 to an area A112, the difference between the average value of the displaced amounts 107 in the first ROI 101 and the average value of the displaced amounts 107 in the second ROIs are out of a predetermined range (-0.01 to 0.01), such that the processings of steps S7 to S9 are successively and repeatedly performed. On the other hand, when the second ROI is an area A113, the difference (0) between the average value (7.33) of the displaced amounts 107 in the first ROI 101 and the average value (7.33) of the displaced amounts 107 in the second ROIs falls within a predetermined range (-0.01 to 0.01) (step S9: Yes), such that the region of interest setting unit 34 sets the area A113, which is the second ROI at that time, as the display region of interest (step S10).

In addition, the elastic image generator 372 generates the data of the elastic image within the display region of interest (step S11), and outputs the data to the display device 4. After step S11, the ultrasound observation apparatus 3 ends a series of processings.

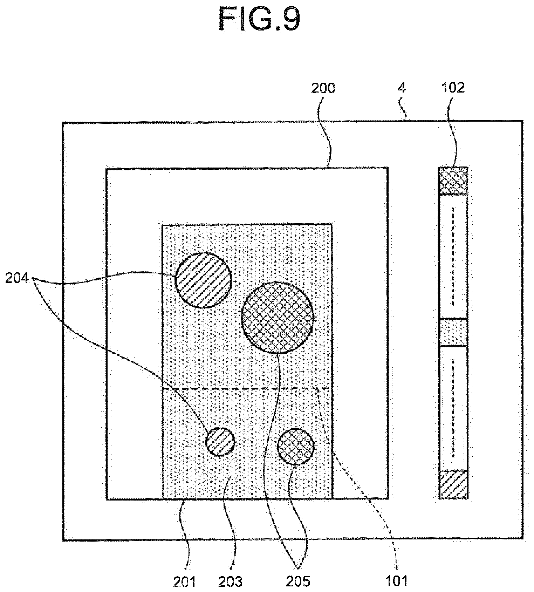

FIG. 9 is a diagram illustrating an example in which the elastic image within the display region of interest is displayed on the display device. In an elastic image 200 illustrated in FIG. 9, an area in which coloring is performed on an image and the hardness of each internal tissue can be visually recognized is expanded from the first ROI 101 indicated by a broken line to a display region of interest 201. An area 203 illustrated in FIG. 9 is an area in which the displaced amount is average. On the other hand, an area 204 is the relatively softest area, and an area 205 is the relatively hardest area.

In this case, when the elastic image generator 372 generates the elastic image, the average value of the displaced amounts in the area in which the elastic image is created is set as a reference value, and the difference between the reference value and the displaced amounts of each pixel 106 becomes large, such that the color of the pixel 106 is determined. As described above, the difference between the average value of the displaced amounts in the first ROI 101 and the average value of the displaced amounts in the display region of interest 201 falls within a predetermined range and is sufficiently small. As a result, the color tone of the first ROI 101 and the area corresponding to the first ROI 101 within the display region of interest 201 remains almost unchanged. Therefore, the ultrasound observation apparatus 3 according to the first embodiment is an ultrasound observation apparatus that can expand the colored area of the elastic image while suppressing the influence on the color tone of the elastic image within the region of interest set by the user in the ultrasound elastography.

It is preferable that the region of interest setting unit 34 sequentially excludes areas farther from the first ROI 101 in order to set the second ROI. There are many cases in which the farther area from the first ROI 101, the larger the deviation of the displaced amount from the reference value that is the average value of the displaced amounts in the first ROI 101, and the display region of interest can be found earlier by preferentially excluding the area.

In addition, above-described first embodiment describes the case of using the ultrasound endoscope 2 including the linear ultrasound transducer 21 in which the sound ray direction is a direction along the vertical direction of the display device 4 and the scanning direction is a direction along the horizontal direction of the display device 4, but is not limited thereto. The ultrasound transducer of the ultrasound endoscope may be a convex type or a radial type, and is not particularly limited as long as it is configured to be able to generate an ultrasound image.

In addition, the predetermined range compared with the difference between the reference value of the first ROI and the reference values of the second ROIs may be determined according to the reference value of the first ROI. For example, the larger the reference value of the first ROI, the wider the predetermined range is set.

In addition, the above-described first embodiment describes the configuration in which the data of the elastic image within the display region of interest are automatically displayed on the display device 4 when the user inputs the freeze instruction signal from the input unit 33, but is not limited thereto. For example, when the user inputs the freeze instruction signal from the input unit 33, a message for allowing the user to select which one of the first ROI and the display region of interest is displayed on the display device 4, and the elastic image generator 372 may be configured to generate the data of the elastic image according to the user selection.

### (Second Embodiment)

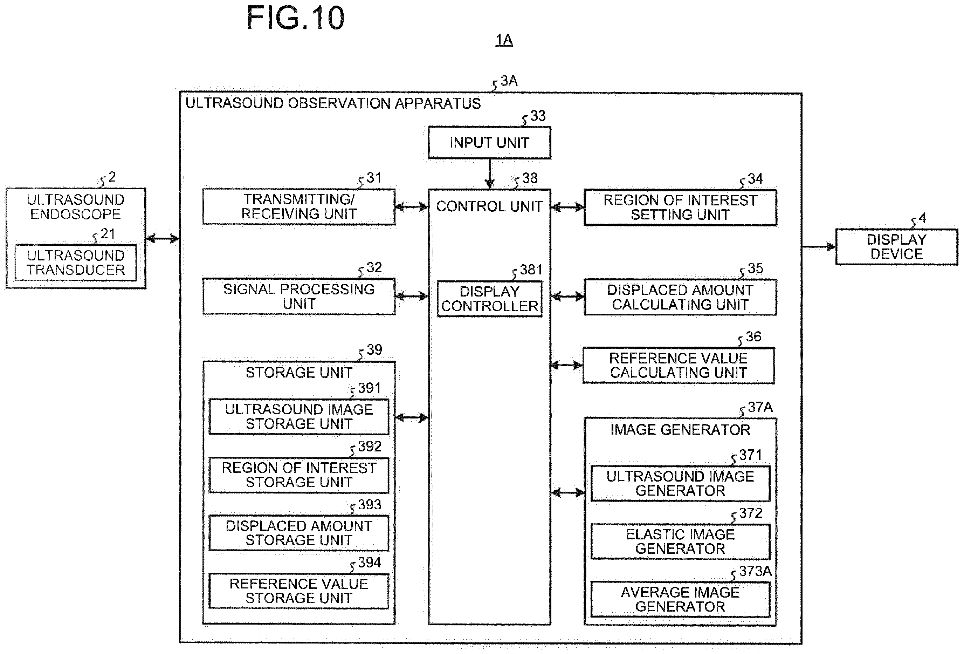

FIG. 10 is a diagram schematically illustrating a configuration of an ultrasound diagnostic system including an ultrasound observation apparatus according to a second embodiment of the present invention. An ultrasound diagnostic system 1A illustrated in FIG. 10 includes an ultrasound endoscope 2, an ultrasound observation apparatus 3A, and a display device 4. A configuration of the ultrasound diagnostic system 1A other than the ultrasound observation apparatus 3A is the same as that of the ultrasound diagnostic system 1 described in the first embodiment.

The ultrasound observation apparatus 3A is different from the ultrasound observation apparatus 3 described in the first embodiment in a configuration of an image generator. An image generator 37A included in the ultrasound observation apparatus 3A includes an average image generator 373A in addition to an ultrasound image generator 371 and an elastic image generator 372.

In a case in which a freeze instruction signal that sets a display on the display device 4 as a still image is input from an input unit 33, when an average instruction signal is further input from the input unit 33, the average image generator 373A adds up and averages pixel values of each pixel in a plurality of ultrasound images composed of designated ultrasound images in the average instruction signal, thereby generating data of an average image. The input unit 33 receives a selective input of an image to be added up and averaged. Specifically, when the input unit 33 receives the input of the average instruction signal, the display controller 381 displays the past ultrasound images to be added up and averaged on the display device 4. In this case, the display controller 381 may display ultrasound images on the display device 4 by one sheet or may display the ultrasound images by several sheets. The average image generator 373A generates average image data using a plurality of ultrasound images selected from the ultrasound images displayed on the display device 4.

The region of interest setting unit 34 sets a first ROI and a plurality of second ROIs in the respective ultrasound images of the plurality of ultrasound images selected. The elastic image generator 372 sets an area, in which the reference value of the first ROI in the respective ultrasound images and the reference values of the second ROIs in the respective ultrasound images have a predetermined correlation and the second ROIs are maximized, in a third region of interest (hereinafter, referred to as a third ROI). In addition, the region of interest setting unit 34 sets an area in an average image corresponding to an area in which each third ROI overlaps with each other as the display region of interest.

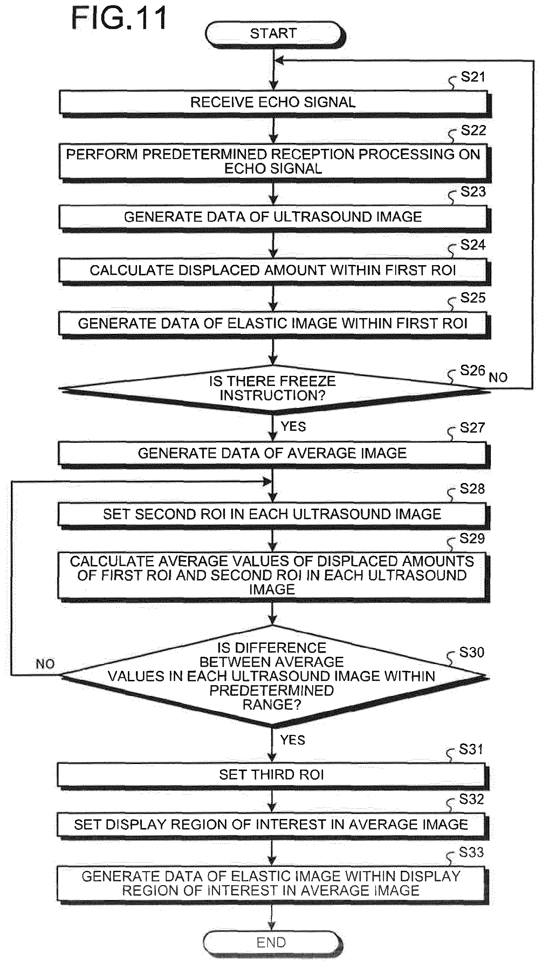

FIG. 11 is a flowchart illustrating an outline of processing performed by the ultrasound observation apparatus according to the second embodiment of the present invention. Even the flowchart illustrated in FIG. 11 illustrates processing in the case in which the ultrasound diagnostic system 1A is set in the elastography mode, the transmitting/receiving unit 31 starts to transmit the transmission drive wave, the ultrasound transducer 21 starts to transmit the ultrasound wave and then the setting of the first ROI in the ultrasound image is completed. Further, it is assumed that the average instruction signal is input in advance from the input unit 33. The processings of steps S21 to S26 sequentially correspond to the processings of steps S1 to S6 described in the first embodiment.

After step S26, the average image generator 373A generates the data of the average image by adding up and averaging pixel values of each pixel in the plurality of ultrasound images composed of the ultrasound images designated in the average instruction signal (step S27). The processings of steps S28 to S30 sequentially correspond to the processings of steps S7 to S9 described in the first embodiment, but these processings are each performed on the respective ultrasound images of the plurality of ultrasound images designated in the averaging instruction signal. As a result, in the respective ultrasound images, the area in which the reference value of the first ROI and the reference values of the second ROIs have a predetermined correlation and the second ROIs are maximized is set in the third ROI (step S31).

Subsequently, the region of interest setting unit 34 extracts the area in which the third ROIs in the respective ultrasound images overlap with each other, and sets the extracted area in the average image as the display region of interest (step S32).

Thereafter, the elastic image generator 372 generates the data of the elastic image within the display region of interest in the average image (step S33), and outputs the data to the display device 4. After step S33, the ultrasound observation apparatus 3 ends a series of processings.

According to the second embodiment of the present invention described above, it is possible to expand the colored area of the elastic image while suppressing the influence on the color tone of the elastic image in the average image.

It should be noted that the input of the average instruction signal to the input unit 33 may be performed at the time of starting a diagnosis by the ultrasound elastography in advance as described above or after the freeze instruction signal is input to the input unit 33.

In addition, in the second embodiment described above, the region of interest setting unit 34 is configured to reliably suppress the influence on the color tone of the elastic image by extracting the area in which the third ROIs in the respective ultrasound images overlap with each other and setting the display region of interest, but is not limited thereto. For example, the region of interest setting unit 34 may also set the area within the average image corresponding to the area set in the third ROI in any of the ultrasound images as the display region of interest. As a result, the display region of interest can be set as a wider area.

In addition, the elastic image generator 372 may set the area, in which the reference value of the first ROI and the reference value of the second ROIs have the predetermined correlation and the second ROIs are maximized, as the display region of interest.

### (Third Embodiment)

A configuration of an ultrasound diagnostic system including an ultrasound observation apparatus according to a third embodiment is the same as the configuration of the ultrasound diagnostic system 1 described in the first embodiment, but is different from the first embodiment in the processing performed by the ultrasound observation apparatus 3.

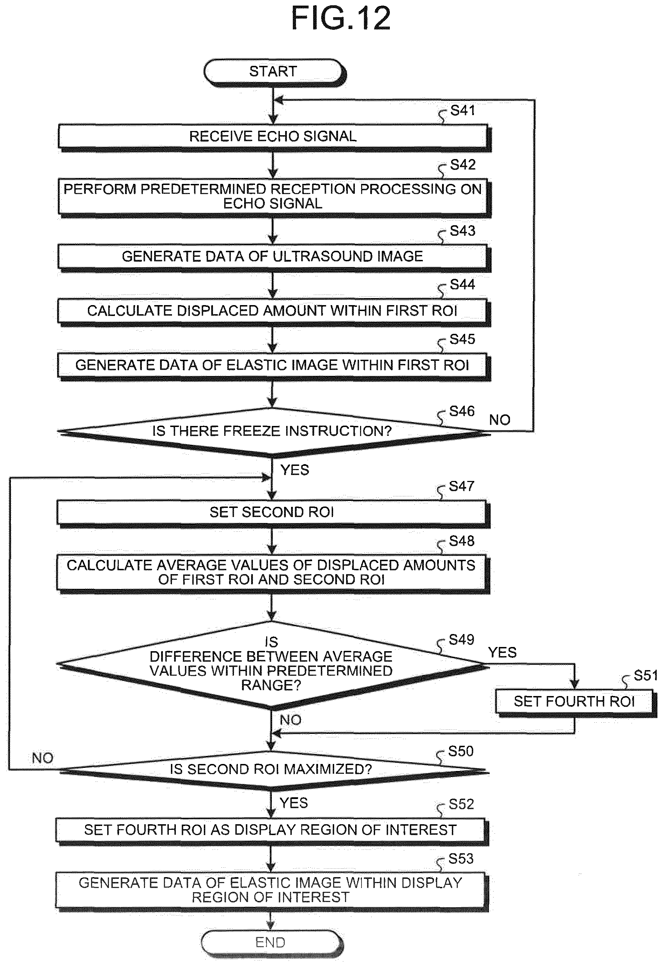

FIG. 12 is a flowchart illustrating an outline of processing performed by the ultrasound observation apparatus according to the third embodiment of the present invention. Even the flowchart illustrated in FIG. 12 illustrates processing in the case in which the ultrasound diagnostic system 1 is set in the elastography mode, the transmitting/receiving unit 31 starts to transmit the transmission drive wave, the ultrasound transducer 21 starts to transmit the ultrasound wave and then the setting of the first ROI in the ultrasound image is completed. Processings of steps S41 to S46 sequentially correspond to the processings of steps S1 to S6 described in the first embodiment.

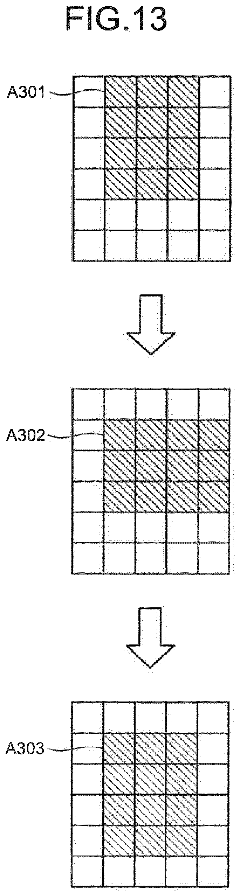

After step S46, the region of interest setting unit 34 sets the second ROIs (step S47). FIGS. 13 to 16 are diagrams illustrating an example of the method for setting a second ROI. As illustrated in FIGS. 13 to 16, the region of interest setting unit 34 expands a part of the area from the first ROI according to a predetermined rule to set the second ROI. Specifically, the region of interest setting unit 34 first sets an area A301, which is obtained by expanding the first ROI upward by one pixel, in the second ROI.

Subsequently, the reference value calculating unit 36 calculates the average value of the displaced amounts in the first ROI and the second ROIs (step S48).

Thereafter, the elastic image generator 372 determines whether the difference between the average value of the displaced amounts in the first ROI and the average value of the displaced amounts in the second ROIs falls within a predetermined range under the control of the display controller 381 (step S49). Specifically, it is determined whether the difference between the average value of the displaced amounts in the first ROI and the average value of the displaced amounts in the second ROI as the area A301 falls within a range of -0.01 to 0.01. When the difference between the average value of the displaced amounts in the first ROI and the average value of the displaced amounts in the second ROIs is larger than 0.01 (step S49: No), the processing proceeds to step S50.

In step 50, it is determined whether or not the area set in the second ROI is the maximum area. As the determination result, when the area set in the second ROI is not the maximum area (step S50: No), the processing returns to step S47 to set new second ROIs. As illustrated in FIG. 13, the new second ROIs are an area A302 and an area 303 obtained by expanding the first ROI to the right side and the lower side, respectively, by one pixel.

In step S49, when the area A303 is set in the second ROI, it is determined that the difference between the average value of the displaced amounts in the first ROI and the average value of the displaced amounts in the second ROIs falls within a range of -0.01 to 0.01 (step S49: Yes). At this time, the region of interest setting unit 34 sets the area A303 in a fourth region of interest (hereinafter, referred to as a fourth ROI) (step S51).

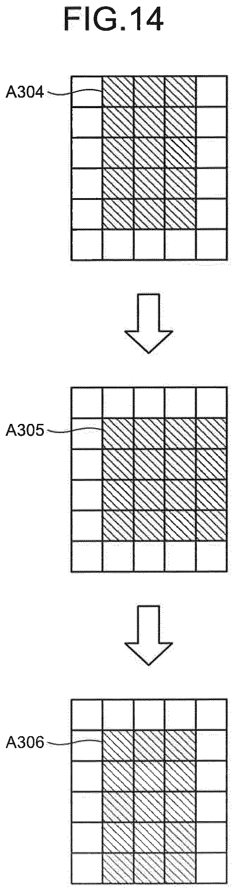

Thereafter, the region of interest setting unit 34 sets the second ROI to expand the area from the fourth ROI. Specifically, as illustrated in FIG. 14, the region of interest setting unit 34 sequentially sets an area A304, an area A305, and an area A306, which are regions obtained by expanding the fourth ROI as the area A303 in up, right, and down directions by one pixel, in the second ROI.

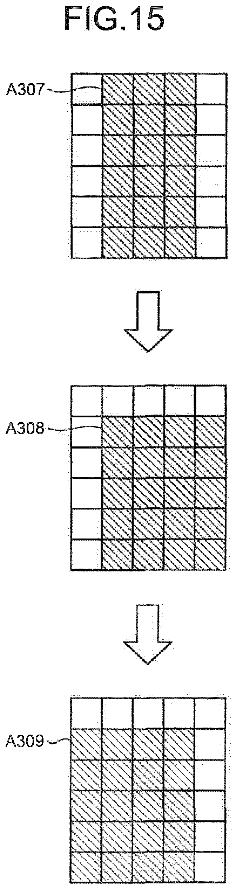

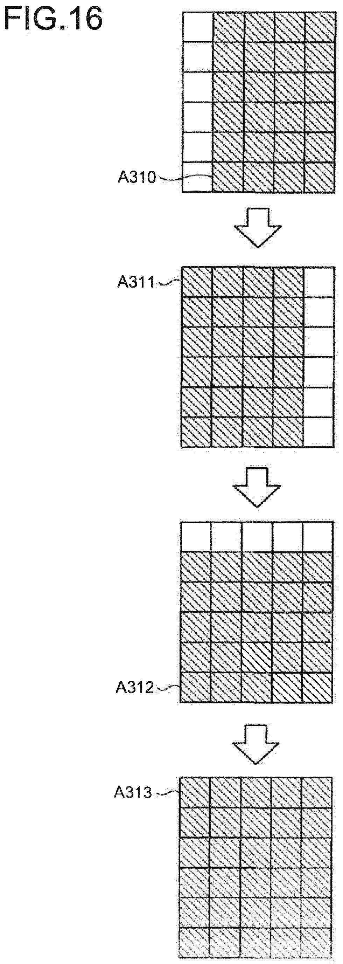

In addition, in step S49, when the area A306 is set in the second ROI, it is determined that the difference between the average value of the displaced amounts in the first ROI and the average value of the displaced amounts in the second ROIs falls within a range of -0.01 to 0.01 (step S49: Yes). At this time, the region of interest setting unit 34 sets the area A306 in a new fourth ROI. Thereafter, as illustrated in FIGS. 15 and 16, the region of interest setting unit 34 sets areas A307 to A313 in the second ROIs so as to expand the area from the fourth ROI.

Thereafter, in step S50, when an area A313 is set in the second ROI, it is determined that the area set in the second ROI is the maximum region (step S50: Yes), and the region of interest setting unit 34 sets the area A306, which is the fourth ROI at that time, as the display region of interest (step S52).

In addition, the elastic image generator 372 generates the data of the elastic image within the display region of interest (step S53), and outputs the data to the display device 4. After step S53, the ultrasound observation apparatus 3 ends a series of processings.

According to the third embodiment of the present invention described above, the region of interest setting unit 34 sets the second ROI so that the area is expanded from the first ROI and sequentially sets the fourth ROI that is larger so that the display region of interest can be found earlier.

In the above-described embodiment, the region of interest setting unit 34 sets the display region of interest while narrowing the second ROI (first embodiment and second embodiment), or sets the display region of interest while expanding the second ROI (third embodiment), but is not limited thereto. For example, the region of interest setting unit 34 may set the second ROIs so as to cover the patterns of all the areas including the first ROI. As a result, the display region of interest is reliably set to be the maximum region.

As an ultrasound probe, for example, a small-diameter ultrasound miniature probe without an optical system may be applied. Ultrasound miniature probes are usually inserted into a biliary tract, a bile duct, a pancreatic duct, a trachea, a bronchus, a urethra, and a ureter, and are used for observing organs (pancreas, lung, prostate, bladder, lymph nodes, and the like) therearound. Further, as the ultrasound probe, an extracorporeal ultrasound probe that is to be irradiated with ultrasound waves from a body surface of the subject may be applied. The extracorporeal ultrasound probe is usually used for observing abdominal organs (liver, gall bladder, and bladder), breast (especially mammary gland), thyroid gland, and the like.

Further effects and variations can be easily derived by those skilled in the art. As a result, the broader aspects of the present invention are not limited to the specific details and representative embodiments shown and described as above. Accordingly, various modifications are possible without departing from the spirit or scope of the general inventive concept as defined by the appended claims and their equivalents.

### Reference Signs List

1, 1A ULTRASOUND DIAGNOSTIC SYSTEM
2 ULTRASOUND ENDOSCOPE
3, 3A ULTRASOUND OBSERVATION APPARATUS
4 DISPLAY DEVICE
21 ULTRASOUND TRANSDUCER
31 TRANSMITTING/RECEIVING UNIT
32 SIGNAL PROCESSING UNIT
33 INPUT UNIT
34 REGION OF INTEREST SETTING UNIT
35 DISPLACED AMOUNT CALCULATING UNIT
36 REFERENCE VALUE CALCULATING UNIT
37, 37A IMAGE GENERATOR
38 CONTROL UNIT
39 STORAGE UNIT
100, 200 ELASTIC IMAGE
101 FIRST ROI
102 COLOR SCALE
103, 104, 105, 203, 204, 205 AREA
106 PIXEL
107 DISPLACED AMOUNT
201 DISPLAY REGION OF INTEREST
371 ULTRASOUND IMAGE GENERATOR
372 ELASTIC IMAGE GENERATOR
373A AVERAGE IMAGE GENERATOR
381 DISPLAY CONTROLLER
391 ULTRASOUND IMAGE STORAGE UNIT
392 REGION OF INTEREST STORAGE UNIT
393 DISPLACED AMOUNT STORAGE UNIT
394 REFERENCE VALUE STORAGE UNIT
A101, A102, A103, A104, A105, A106, A107, A108, A109, A110, A111, A112, A113, A301, A302, A303, A304, A305, A306, A307, A308, A309, A310, A311, A312, A313 AREA

## Claims

1. An ultrasound observation apparatus performing an observation based on an ultrasound wave signal acquired by an ultrasound probe including an ultrasound transducer transmitting an ultrasound wave to an observation object and receiving the ultrasound wave reflected from the observation object, the ultrasound observation apparatus comprising:
an ultrasound image generator configured to generate data of an ultrasound image based on the ultrasound wave signal;
a region of interest setting unit configured to set a first region of interest set in advance within the ultrasound image and a plurality of second regions of interest including at least the first region of interest, when receiving an input of a freeze instruction signal that sets a display on a display device as a still image;
a reference value calculating unit configured to calculate reference values according to ultrasound wave signals of the first region of interest and the plurality of second regions of interest, respectively; and
an elastic image generator configured to set a display region of interest in which the reference value of the first region of interest and the reference values of the second regions of interest have a predetermined correlation and the second regions of interest are maximized, and generate data of an elastic image having a display mode according to hardness of the display region of interest.

2. The ultrasound observation apparatus according to claim 1, wherein the predetermined correlation is a relationship in which a ratio or a difference between the reference value of the first region of interest and the reference values of the second regions of interest falls within a predetermined range.

3. The ultrasound observation apparatus according to claim 1 or 2, wherein
the region of interest setting unit is configured to successively set the second regions of interest by excluding a part of the second regions of interest having a maximum area within the ultrasound image from the second regions of interest having the maximum area within the ultrasound image according to a predetermined rule,
the reference value calculating unit is configured to successively calculate the reference values of the second regions of interest that the region of interest setting unit has set, and
the elastic image generator is configured to successively compare the reference value of the first region of interest with the reference values of the second regions of interest, and when the reference value of the first region of interest and the reference values of the second regions of interest have the predetermined correlation, set the second regions of interest at that time as the display region of interest.

4. The ultrasound observation apparatus according to claim 3, wherein the region of interest setting unit is configured to set the second regions of interest by sequentially excluding an area farther from the first region of interest.

5. The ultrasound observation apparatus according to claim 3 or 4, wherein the region of interest setting unit is configured to set the second regions of interest by sequentially excluding an area along a sound ray of the ultrasound transducer constituting the ultrasound image and an area along a scanning direction of the ultrasound transducer constituting the ultrasound image.

6. The ultrasound observation apparatus according to any one of claims 1 to 5, further comprising:
an average image generator configured to generate, when receiving an input of an average instruction signal for generating an average image obtained by adding up and averaging a plurality of ultrasound images, data of the average image,
wherein the region of interest setting unit is configured to set the first region of interest and the plurality of second regions of interest in the respective ultrasound images of the plurality of ultrasound images, and
the elastic image generator is configured to set an area, in which the reference value of the first region of interest in the respective ultrasound images and the reference values of the second regions of interest in the respective ultrasound images have the predetermined correlation and the second regions of interest are maximized, as third regions of interest, and set an area within the average image corresponding to an area in which the respective third regions of interest overlap with each other, as the display region of interest.

7. The ultrasound observation apparatus according to claim 1, wherein the region of interest setting unit is configured to set the second regions of interest obtained by expanding a part of the first region of interest from the first region of interest according to a predetermined rule.

8. The ultrasound observation apparatus according to any one of claims 1 to 7, wherein
the predetermined correlation is a relationship in which a difference between the reference value of the first region of interest and the reference values of the second regions of interest falls within a predetermined range, and
the elastic image generator is configured to change the predetermined range according to the reference value of the first region of interest.

9. The ultrasound observation apparatus according to any one of claims 1 to 8, wherein the reference value is a statistic corresponding to the ultrasound wave signal.

10. An operation method for an ultrasound observation apparatus performing an observation based on an ultrasound wave signal acquired by an ultrasound probe including an ultrasound transducer transmitting an ultrasound wave to an observation object and receiving the ultrasound wave reflected from the observation object, the operation method comprising:
a region of interest setting step of setting, by a region of interest setting unit, a first region of interest set in advance within an ultrasound image generated based on the ultrasound wave signal and a plurality of second regions of interest including at least the first region of interest, when an input of a freeze instruction signal that sets a display on a display device as a still image is received;
a reference value calculation step of calculating, by a reference value calculating unit, reference values according to ultrasound wave signals of the first region of interest and the plurality of second regions of interest, respectively; and
an elastic image generation step of setting, by an elastic image generator, a display region of interest in which the reference value of the first region of interest and the reference values of the second regions of interest have a predetermined correlation and the second regions of interest are maximized, and generating data of an elastic image having a display mode according to hardness of the display region of interest.

11. An operation program for an ultrasound observation apparatus causing the ultrasound observation apparatus performing an observation based on an ultrasound wave signal acquired by an ultrasound probe including an ultrasound transducer transmitting an ultrasound wave to an observation object and receiving the ultrasound wave reflected from the observation object to execute the following steps:
a region of interest setting step of setting, by a region of interest setting unit, a first region of interest set in advance within an ultrasound image generated based on the ultrasound wave signal and a plurality of second regions of interest including at least the first region of interest, when an input of a freeze instruction signal that sets a display on a display device as a still image is received;
a reference value calculation step of calculating, by a reference value calculating unit, reference values according to ultrasound wave signals of the first region of interest and the plurality of second regions of interest, respectively; and
an elastic image generation step of setting, by an elastic image generator, a display region of interest in which the reference value of the first region of interest and the reference values of the second regions of interest have a predetermined correlation and the second regions of interest are maximized, and generating data of an elastic image having a display mode according to hardness of the display region of interest.
